# FASCICULE DE BREVET EUROPEEN

(11) **EP 4 046 914 B1**
(45) Date de publication et mention de la délivrance du brevet: **25.10.2023**
(21) Numéro de dépôt: 22157809.9
(22) Date de dépôt: 21.02.2022
(51) Int. Cl.: B64D 45/00, G08B 21/06, A61B 5/18

(54) **SYSTÈME DE SURVEILLANCE DE L ÉTAT OPÉRATIONNEL D'UN ÉQUIPAGE D AÉRONEF, ET PROCÉDÉ ASSOCIÉ**
SYSTEM ZUR ÜBERWACHUNG DES EINSATZZUSTANDS EINER FLUGZEUGBESATZUNG UND ENTSPRECHENDES VERFAHREN
SYSTEM FOR MONITORING THE WORKING CONDITION OF AN AIRCRAFT CREW, AND ASSOCIATED METHOD

(30) Priorité: 22.02.2021 FR 2101673
(43) Date de publication de la demande: 24.08.2022
(73) Titulaire: Dassault Aviation, 75008 Paris (FR)
(72) Inventeur: SALMON-LEGAGNEUR, François, 92214 SAINT CLOUD (FR); LIGIER, Valentin, 92214 SAINT-CLOUD (FR); SAINT REQUIER, Cyril, 92214 SAINT CLOUD (FR)
(74) Mandataire: Lavoix

(56) Documents cités:
- EP-A1- 3 154 038
- US-A1- 2006 220 883
- LIU JING ET AL: "Cognitive pilot-aircraft interface for single-pilot operations", KNOWLEDGE-BASED SYSTEMS, ELSEVIER, AMSTERDAM, NL, vol. 112, 6 septembre 2016 (2016-09-06), pages 37-53, XP029757287, ISSN: 0950-7051, DOI: 10.1016/J.KNOSYS.2016.08.031

## Description

La présente invention concerne un système de surveillance de l'état opérationnel d'un équipage d'aéronef, selon le préambule de la revendication 1.

Un tel système est destiné notamment à être intégré à l'avionique d'un aéronef pour surveiller l'état opérationnel d'au moins un membre d'équipage de l'aéronef, en particulier pour déterminer des états dégradés pouvant perturber ou mettre en danger la conduite de l'aéronef. Le système surveille indépendamment et simultanément chaque pilote à poste, ou en variante, seul l'un des pilotes à poste.

Les états dégradés sont par exemple une absence du membre d'équipage hors du cockpit, une incapacité totale ou partielle du membre d'équipage, un état de somnolence, un état d'inattention, un état de surcharge de travail ou/et un état d'engagement excessif dans une tâche.

L'état opérationnel de chaque pilote, c'est-à-dire sa capacité à opérer l'aéronef de manière fiable et en toute conscience, est un paramètre important pour assurer la sécurité des vols. En effet, des défauts d'inattention de chaque pilote sous l'effet de la fatigue ou d'états de stress, et dans le pire des cas, une incapacité totale ou partielle du pilote, ou son absence du cockpit peuvent engendrer des risques substantiels dans la conduite du vol, voire même des accidents.

Généralement, chaque pilote à poste s'assure de l'état opérationnel de l'autre pilote à poste, en surveillant ses réactions, et en contrôlant ses actions.

Cependant, dans certains cas, il peut être utile de compléter l'action de surveillance effectuée par chaque pilote sur l'autre pilote par des moyens automatiques permettant de détecter objectivement des états dégradés d'un membre de l'équipage.

À cet effet, des systèmes de surveillance de l'état opérationnel d'un équipage d'aéronef existent. Par exemple, US10426393 décrit un système de surveillance de la santé d'un pilote, utilisant une pluralité de capteurs qui mesurent des paramètres physiologiques du pilote, et/ou qui suivent les actions du pilote.

Un autre exemple de l'art antérieur est fourni par le document US2006220883.

Les systèmes de surveillance actuels sont fondés sur des capteurs dont la fiabilité ne correspond pas nécessairement aux normes strictes s'appliquant dans le domaine aéronautique.

Par ailleurs, ces systèmes de surveillance utilisent parfois des modules d'intelligence artificielle qui sont capables dans certains cas de détecter des états dégradés par apprentissage. Ces systèmes peuvent fournir par contre des résultats erronés dans d'autres cas non prédictibles, provoquant des fausses alertes sur l'état dégradé du pilote surveillé.

Ce type de système de surveillance est donc généralement une aide, ou tout du moins un filet de sécurité supplémentaire pour les pilotes, mais sa fiabilité ne correspond pas à celle qui est requise dans le domaine aéronautique, notamment pour obtenir une certification auprès des autorités compétentes.

Un but de l'invention est donc de fournir un système de surveillance de l'état opérationnel d'un équipage d'aéronef, qui permette d'obtenir un suivi à la fois très fiable et réactif de l'état d'au moins un membre d'équipage, ce système étant de préférence apte à répondre aux exigences de certification.

À cet effet, l'invention a pour objet un système selon la revendication 1.

Le niveau d'assurance de conception (« Design Assurance Level » ou « DAL ») est généralement déterminé pour un système particulier produisant des données par des études de sécurité de fonctionnement et par une analyse des risques examinant les conséquences d'une condition de défaut ou de panne du système.

Les niveaux possibles d'assurance de conception sont par exemple définis dans la norme ARP4754. Ils sont par exemple désignés par des lettres, comme suit :
Niveau A : Un défaut du système ou sous-système étudié peut provoquer un problème catastrophique - Sécurité du vol ou atterrissage compromis - Crash de l'avion (le niveau A requiert par exemple un taux d'occurrence de défaut ou de panne inférieur ou égal à 10⁻⁹/h de vol) ;
Niveau B : Un défaut du système ou sous-système étudié peut provoquer un problème dangereux entraînant des dégâts sérieux voire la mort de quelques occupants (le niveau B requiert par exemple un taux d'occurrence de défaut ou de panne inférieur ou égal à 10⁻⁷/h de vol) ;
Niveau C : Un défaut du système ou sous-système étudié peut provoquer un problème majeur entraînant un dysfonctionnement des équipements vitaux de l'appareil (le niveau C requiert par exemple un taux d'occurrence de défaut ou de panne inférieur ou égal à 10⁻⁵/ h de vol) ;
Niveau D : Un défaut du système ou sous-système étudié peut provoquer un problème mineur sans effet sur la sécurité du vol (le niveau D requiert par exemple un taux d'occurrence de défaut ou de panne inférieur ou égal à 10⁻³/ h de vol) ;
Niveau E : Un défaut du système ou sous-système étudié peut provoquer un problème sans effet sur la sécurité du vol.

Les termes « haut niveau d'assurance de conception » et « plus bas niveau d'assurance de conception » s'entendent de manière relative en fonction du niveau d'assurance de conception du système engendrant les données. Par exemple, si les niveaux A, B, ou C sont considérés dans une mise en oeuvre particulière de l'invention comme des « hauts niveaux d'assurance de conception », les niveaux D et E sont alors considérés comme des « plus bas niveaux d'assurance de conception ».

Le système selon l'invention peut aussi comprendre l'une ou plusieurs des caractéristiques des revendications 2 à 13 ou l'une des caractéristiques suivantes
- le système comporte un afficheur, et un gestionnaire d'affichage sur l'afficheur, propre à générer et à afficher sur l'afficheur au moins une fenêtre de suivi de l'état de fonctionnement et de désactivation sélective du ou de chaque capteur de surveillance d'états du membre d'équipage, de réglage du niveau de sensibilité de détection de chaque capteur de surveillance d'états du membre d'équipage, de réglage du niveau d'information ou/et d'alarme, ou/et de mise en silencieux ou d'extinction du système de suivi ;
- pour le ou chaque état pilote surveillé, le premier module d'évaluation de haut niveau détermine un état pilote de haut niveau associé à l'état pilote surveillé, le deuxième module d'évaluation de bas niveau détermine un état pilote de bas niveau associé à l'état pilote surveillé, l'état pilote surveillé étant obtenu à partir de l'état pilote consolidé obtenu par le module de consolidation ou étant l'état pilote consolidé obtenu par le module de consolidation.

L'invention a également pour objet un procédé de surveillance de l'état opérationnel d'un équipage d'aéronef selon la revendication 14. Le procédé selon l'invention peut aussi comprendre les pas de procédé de la revendication 15.

L'invention sera mieux comprise à la lecture de la description qui va suivre, donnée uniquement à titre d'exemple, et faite en se référant aux dessins annexés, sur lesquels :
- La figure 1 est un schéma synoptique illustrant un système de surveillance de l'état opérationnel d'un équipage d'aéronef selon l'invention, intégré au sein de l'avionique de l'aéronef ;
- La figure 2 est un schéma synoptique illustrant plus en détails l'unité de détermination de l'état pilote du système de surveillance de la figure 1, avec des modules d'évaluation de haut niveau et de plus bas niveau ;
- La figure 3 illustre un exemple de machine à états finis utilisée par le module d'évaluation de haut niveau pour déterminer un état d'absence d'un membre d'équipage, à l'aide de données de surveillance de haut niveau de fiabilité de conception ;
- La figure 4 illustre une machine à états finis utilisée par le module d'évaluation bas niveau, permettant de déterminer l'absence d'un membre d'équipage à son siège, à l'aide de données de surveillance de plus bas niveau de fiabilité de conception
- Les figures 5 à 7 représentent des fenêtres illustratives de l'interface de présentation, en particulier du ou des états pilote suivis par le système de la figure 1, de l'historique des états pilote, et de commande de désactivation du module d'évaluation bas niveau ; et
- La figure 8 illustre une fenêtre d'alerte propre à s'afficher, lorsque le système de surveillance détecte un état d'absence du pilote dans le cockpit.

Les figures décrites ci-dessus illustrent des fenêtres réelles propres à s'afficher dans un aéronef, et comprennent donc des indications en anglais, conformément aux conventions dans le domaine aéronautique. Si nécessaire, une traduction en français des indications de ces fenêtres est donnée dans la description ci-dessous.

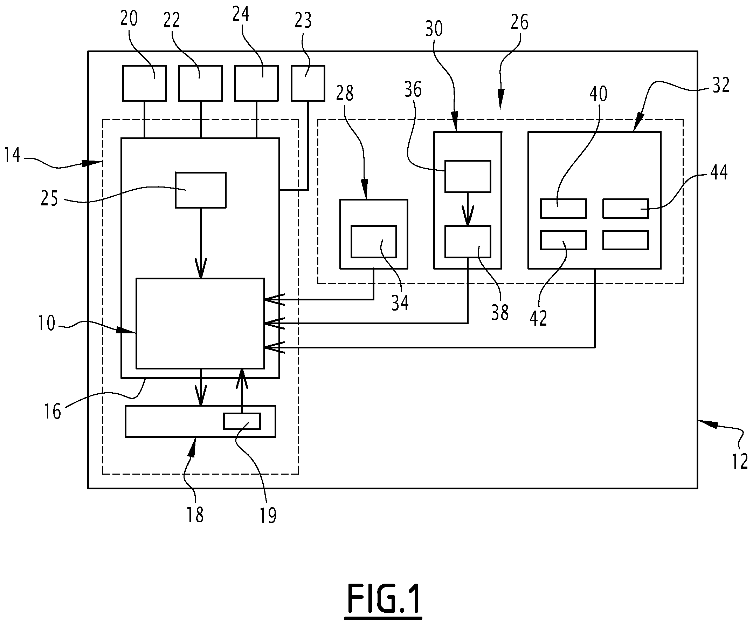

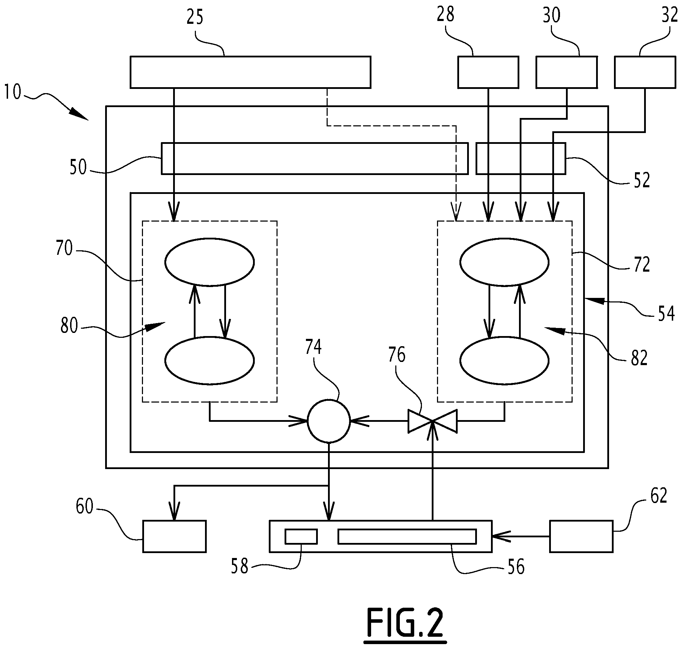

Un premier système 10 de surveillance de l'état opérationnel d'un équipage d'un aéronef 12 est illustré schématiquement sur les figures 1 et 2.

Le système 10 est destiné à être relié à ou à être intégré à un système central d'avionique 14 comportant une unité centrale d'avionique 16, au moins une unité d'affichage 18 placée dans une interface de contrôle de l'aéronef 12 et au moins une interface homme-machine 19 permettant à l'équipage d'interagir avec l'unité centrale d'avionique 14, notamment pour commander les systèmes fonctionnels de l'aéronef 12.

L'interface de contrôle de l'aéronef 12 est par exemple située dans l'aéronef 12 lui-même (dans un cockpit), ou dans une salle de commande à distance de l'aéronef 12 (appelée « station sol »).

L'unité centrale d'avionique 16 est notamment raccordée à des équipements de l'aéronef 12, composant des systèmes fonctionnels de l'aéronef.

Les systèmes fonctionnels de l'aéronef 12 incluent par exemple des systèmes 20 de mesure de l'état de l'aéronef, des systèmes 22 de communication extérieure, des systèmes 24 d'actionnement des commandes de l'aéronef et des systèmes 23 de navigation et de gestion de la mission opérationnelle.

Les systèmes de mesure 20 comportent par exemple des composants comprenant des capteurs de mesure de paramètres extérieurs à l'aéronef, tels que la température, la pression ou la vitesse, des capteurs de mesure de paramètres internes à l'aéronef et à ses différents systèmes fonctionnels et des capteurs de positionnement, tels que des capteurs GPS, des centrales à inertie, et/ou un altimètre.

Les systèmes de communication extérieure 22 incluent par exemple des composants comprenant des systèmes radio, VOR/LOC, ADS, DME, ILS, des systèmes radars, et/ou des systèmes de communication par satellite.

Les systèmes de commande 24 incluent des composants comprenant des actionneurs propres à actionner des commandes de l'aéronef, tels que des volets, des gouvernes, des pompes, ou encore des circuits mécaniques, électriques ou/et hydrauliques, et des actionneurs logiciels propres à configurer les états avioniques de l'aéronef.

Les systèmes 23 de navigation et de gestion de la mission opérationnelle comprennent par exemple un système de gestion de vol (« Flight Management System » en anglais), et éventuellement un système de gestion de tâches de mission. Ces systèmes sont propres à identifier la phase de vol et à aider les pilotes dans la navigation de l'aéronef suivant une trajectoire, et dans la gestion des tâches associées à la navigation. Ils sont propres à produire des données utilisables par le système de suivi 10 telles que l'identification de la phase de vol courante, l'altitude de l'avion, l'heure du jour, etc.

Les différents systèmes 20 à 24 sont raccordés à l'unité centrale d'avionique 14, par exemple de manière numérique, par au moins un bus de données 25 circulant sur un réseau interne à l'aéronef 12.

Le bus de données 25 est apte à transporter notamment les données requises par le système 10 pour fonctionner.

Ces données sont par exemple des données d'avionique, en particulier des données d'activation de commande sur l'interface 19, des données de dispositif de pointage et désignation par curseur (« Cursor Control Device ou CCD en anglais) pour piloter l'interface 19 ou d'autres données d'interaction d'utilisateurs avec l'interface 19, notamment sur les afficheurs 18 de l'aéronef.

Lorsqu'au moins un membre de l'équipage effectue des actions d'interaction avec le système central d'avionique 14, par exemple agit sur les commandes, ou effectue des interactions avec des dispositifs de pointage et désignation par curseur, ou avec des écrans tactiles de l'aéronef, des premières données de haut niveau d'assurance de conception sont engendrées. Les premières données de haut niveau d'assurance de conception ainsi engendrées permettent généralement d'identifier quel membre d'équipage effectue ces actions d'interaction.

Le système de surveillance 10 est également raccordé à un système 26 de capteurs de surveillance équipage, non intégré dans le système central d'avionique 14. Les capteurs du système de capteurs de surveillance équipage 26 produisent ici des données, qui peuvent être des données de haut niveau d'assurance de conception et/ou des données de plus bas niveau d'assurance de conception, et qui peuvent également être transportées par le bus de données 25.

Comme indiqué plus haut, le niveau d'assurance de conception des données est par exemple défini par la Norme ARP4754.

Dans l'exemple illustré sur la figure 1, le système de capteurs 26 inclut avantageusement un capteur 28 de détection de présence sur un siège, disposé sur un siège destiné à recevoir un membre d'équipage dans l'interface de commande de l'aéronef, un système 30 de caméra et/ou au moins un capteur additionnel 32.

Le capteur 28 de détection de présence sur un siège comprend par exemple une jauge de pression 34, qui est apte à détecter qu'un membre de l'équipage exerce une pression sur l'assise ou/et sur le dossier du siège.

Le système de caméra 30 comporte au moins une caméra 36, et un dispositif d'analyse 38 des images produites par la caméra 36, pour déterminer des données de présence et de mouvement d'un membre de l'équipage présent dans l'interface de commande de l'aéronef, ainsi qu'une direction de vision du membre d'équipage et avantageusement d'autres paramètres physiologiques et cognitifs associés au membre d'équipage (comme la somnolence, la distraction, ...).

La caméra 36 est par exemple une caméra fonctionnant dans le domaine visible, proche infrarouge, thermique, et/ou une caméra temps de vol, en 2D et/ou en 3D.

Les capteurs additionnels 32 comportent par exemple un capteur 40 de mesure du rythme cardiaque d'un membre d'équipage, en particulier un capteur d'électrocardiographie (ECG), des capteurs 42 de mesure d'activité mentale du membre d'équipage, en particulier des capteurs fonctionnels de proche infrarouges (FNIR) ou des capteurs d'électroencéphalographie (EEG). Les capteurs additionnels 32 incluent avantageusement des capteurs de poignet, tels que des montres connectées 44 ou d'autres capteurs divers pouvant être interfacés avec le système de surveillance 10 au sein du système de capteurs de surveillance d'équipage 26.

En référence à la figure 2, le système de surveillance de l'état opérationnel d'un équipage 10 comporte une première interface 50 de réception exclusive de premières données de surveillance d'équipage présentant un haut niveau d'assurance de conception, et une deuxième interface 52 de réception de deuxièmes données de surveillance d'équipage présentant un niveau d'assurance de conception de plus bas niveau.

Le système de surveillance de l'état opérationnel d'un équipage 10 comporte en outre une unité 54 de détermination d'au moins un état pilote en fonction des premières données de surveillance reçues de la première interface de réception 50 et des deuxièmes données de surveillance reçues de la deuxième interface 52. Il comporte également un afficheur 56 et un gestionnaire d'affichage 58 sur l'afficheur 56, propre à afficher au moins une fenêtre de surveillance d'équipage.

Le système de surveillance de l'état opérationnel d'un équipage 10 comporte en outre avantageusement un générateur d'informations et/ou d'alarme 60, et au moins une interface homme/machine 62 pour permettre à l'équipage d'interagir avec le système de surveillance de l'état opérationnel d'un équipage 10.

L'unité de détermination d'état pilote 54 est propre à déterminer un état pilote, ou de préférence, à déterminer plusieurs états pilotes en parallèle.

L'état pilote est par exemple un état de présence à son poste de travail, un état de capacité d'opération de l'aéronef, en particulier d'incapacité ou/et de sommeil, un état de vigilance, notamment de somnolence, de distraction, ou d'inattention, un état de surcharge de travail, un état de surcharge mentale, un état de stress, un état de niveau de conscience de la situation présente, un état d'engagement dans une tâche, un état de niveau d'activité physique, et/ou un état de niveau de cohérence et de pertinence de l'activité pilote avec la mission.

L'unité de détermination d'état pilote 54 comporte par exemple un processeur, et au moins une mémoire destinée à recevoir des modules logiciels propres à être exécutés par le processeur pour effectuer des fonctions.

En variante, l'unité de détermination d'état pilote 54 comporte des composants logiques programmables ou des circuits intégrés dédiés, destinés à réaliser les fonctions des modules qui seront décrits ci-après.

En référence à la figure 2, l'unité de détermination d'état pilote 54 comporte, pour le ou pour chaque état pilote surveillé, un premier module d'évaluation 70 des premières données de haut niveau d'assurance de conception, propre à mettre en oeuvre un algorithme déterministe pour obtenir un état pilote de haut niveau, à partir des seules premières données de haut niveau d'assurance de conception, sans tenir compte des deuxièmes données de plus bas niveau d'assurance de conception.

L'unité de détermination d'état pilote 54 comporte en outre, pour le ou pour chaque état pilote surveillé, un deuxième module 72 d'évaluation de plus bas niveau, propre à déterminer un état pilote de bas niveau, à partir notamment des deuxièmes données de plus bas niveau d'assurance de conception.

L'unité de détermination d'état pilote 54 comporte également, pour le ou pour chaque état pilote surveillé, un module de consolidation 74, propre à déterminer, dans une configuration active, un état pilote consolidé sur la base de l'état pilote de haut niveau et de l'état pilote de bas niveau. Elle comporte en outre une commande 76 propre à basculer le module de consolidation 74 dans une configuration inactive du deuxième module d'évaluation 72 pour que le module de consolidation 74 détermine l'état pilote sur la base de l'état pilote de haut niveau uniquement.

Le ou chaque état pilote déterminé par le module de consolidation 74 est un état choisi parmi un état normal et au moins un état dégradé.

L'état dégradé est par exemple déterminé parmi un état d'absence du pilote hors de son poste de travail, un état d'incapacité ou de sommeil du pilote empêchant le pilote d'opérer l'aéronef, un état de somnolence légère ou importante, un état de perte de vigilance, un état de surcharge de travail ou/et un état d'engagement excessif dans une tâche. D'autres états dégradés peuvent être définis en fonction des capteurs présents dans le système de capteurs 26.

Pour chaque état pilote, un état normal correspond à l'état dégradé. L'état normal est par exemple un état de présence du pilote à son poste, un état de capacité ou d'éveil du pilote lui permettant d'opérer l'aéronef, un état d'alerte, un état de vigilance, un état de charge de travail normale ou/et un état d'engagement normal dans une tâche.

Les états d'absence, d'incapacité et/ou de sommeil, dans lesquels le pilote est inapte à se déclarer via l'interface 19 après une sollicitation, sont susceptibles d'engendrer des informations, par exemple des messages ou des notifications, voire éventuellement des alertes par le générateur d'informations et/ou d'alarme 60.

À titre d'exemple, l'état d'absence peut être détecté à partir des premières données de haut niveau, en l'absence d'activité sur les commandes avioniques par un membre d'équipage pendant une durée prédéfinie (par exemple 5 minutes) et de manière cumulative, lorsqu'aucune réaction du pilote n'est observée en réaction à une fenêtre de sollicitation émise au niveau d'un afficheur 18 du système d'avionique 14.

L'état d'absence peut également être détecté par l'absence de pression sur le siège exercée par le membre d'équipage pendant une durée prédéfinie (par exemple 30 secondes), détectée par la jauge de pression 34 du capteur de détection de présence 28, ou par l'absence de reconnaissance de visage effectuée par le dispositif d'analyse d'images 38 couplé à la caméra 36 du capteur du système de caméra 30, pendant une durée prédéfinie (par exemple comprise 1 minute).

L'état d'incapacité et/ou de sommeil peut être détecté par l'absence d'activité pilote sur les commandes d'avionique pendant une durée prédéfinie (par exemple 5 minutes), et de manière cumulative en l'absence de réaction du pilote à une fenêtre de sollicitation après une durée prédéfinie (par exemple 30 secondes).

Généralement, l'incapacité et/ou le sommeil ne sont pas détectables au niveau de la jauge de pression 34 du capteur de position siège 28 qui détectent bien la présence du membre d'équipage. Par contre, l'incapacité et/ou le sommeil sont également détectables par le dispositif d'analyse d'images 38 lié à la caméra 36 du système de caméra 30 en l'absence de mouvement du pilote, ou par la posture détectée du corps du pilote.

L'état de somnolence est par exemple détecté en mesurant à l'aide de la caméra 36 et du dispositif d'analyse d'images 38 l'activité oculaire du pilote assis.

L'état d'inattention est mesuré à l'aide du système de caméra 30, en mesurant la position du regard de l'utilisateur, notamment si cette position est éloignée des positions que l'utilisateur devrait regarder en regard des interfaces ou du cockpit, par exemple parce qu'il consulte un téléphone portable ou une tablette. En outre, ceci est corroboré avec un manque d'activité du pilote sur les commandes, et éventuellement en l'absence de réaction du pilote à une fenêtre de sollicitation.

Les états dégradés d'absence et d'inactivité (incapacité et/ou de sommeil), sont destinés à être détectés au moins par le module d'évaluation de haut niveau 70. Ils sont propres à être détectés en parallèle par le module d'évaluation de plus bas niveau 72.

Dans cet exemple, les états dégradés de somnolence et/ou d'inattention sont propres à être détectés principalement par le module 72 d'évaluation de plus bas niveau.

Le premier module d'évaluation de haut niveau 70 est raccordé à la première interface 50, pour recevoir exclusivement des premières données de surveillance de haut niveau d'assurance de conception.

Il comporte au moins une première machine à états 80, propre à mettre en oeuvre un algorithme déterministe sur la base des premières données de surveillance de haut niveau d'assurance de conception. La première machine à états 80 est destinée à déterminer l'état pilote de haut niveau, entre un état normal et un état dégradé tel que défini plus haut.

Préférentiellement, le premier module d'évaluation de haut niveau 70 présente une machine à états 80 pour chaque état pilote à déterminer, entre l'état pilote normal et l'état pilote dégradé. Il comprend donc ici au moins une machine à états 80 pour l'état d'absence et au moins une machine à états 80 pour l'état d'incapacité ou/et de sommeil.

Le premier module d'évaluation 70 est donc propre, pour le ou chaque état pilote surveillé par l'unité de détermination 54, à obtenir un état pilote de haut niveau, choisi entre un état pilote normal de haut niveau et un état pilote dégradé de haut niveau.

La machine à états 80 met en oeuvre un algorithme déterministe, c'est-à-dire un algorithme qui, en réponse à un jeu de premières données de surveillance, donne toujours le même résultat.

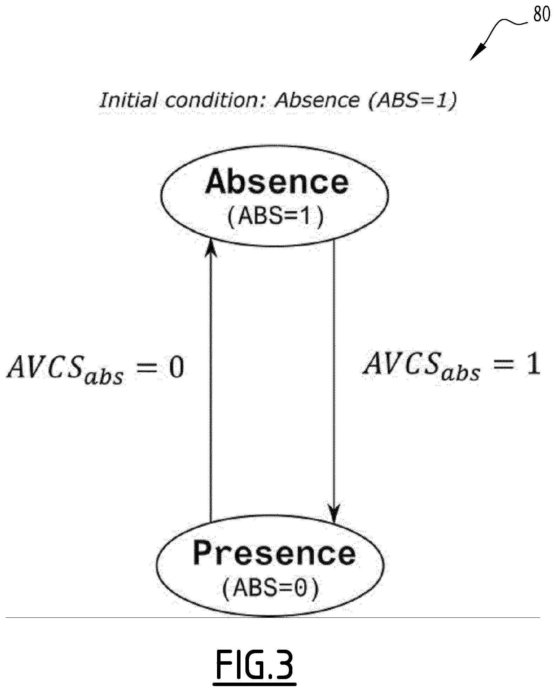

Un exemple de machine à états 80 pour la détermination d'un état d'absence est illustré sur la figure 3. La machine à états 80 est propre à déterminer un état d'absence ou un état de présence du pilote dans l'interface de commande sous forme d'un indicateur booléen ici désigné par ABS. Par défaut, l'état détecté est un état initial d'absence (« initial condition : Absence (ABS = 1) » ; « la condition initiale : Absence (ABS = 1) »).

La machine à états 80 est propre à récupérer des premières données de haut niveau à partir de l'interface 50 pour déterminer un indicateur booléen de détection du pilote AVCS_{abs}. L'indicateur présente une valeur égale à 1 lorsque par exemple les premières données de surveillance indiquent des actions d'interaction du pilote avec le système central d'avionique 14 au cours du passé récent.

L'indicateur de présence est élaboré au sein de l'unité de détermination de l'état pilote 54 à partir de l'ensemble des données reçues au travers de l'interface 50, ces données ne pouvant varier que sur action du pilote, ils forment ainsi un indicateur de présence et d'activité humaine dans le cockpit.

Dans ce cas, la condition de transition étant vraie, l'état d'absence passe à une valeur nulle et le pilote est détecté comme présent. L'état pilote de haut niveau est alors un état pilote de haut niveau normal.

Au contraire, si l'indicateur booléen de détection du pilote AVCS_{abs} est nul, c'est-à-dire que le pilote n'a pas agi sur les commandes pendant une durée prédéfinie, la machine à états 80 repasse dans l'état d'absence qui correspond à un état dégradé de haut niveau.

Dans cet exemple, le deuxième module d'évaluation de plus bas niveau 72 est propre à recevoir des deuxièmes données de surveillance de plus bas niveau d'assurance de conception provenant de la deuxième interface 52, et avantageusement également des premières données de surveillance de haut niveau d'assurance de conception, provenant de la première interface 50.

Le deuxième module d'évaluation 72 est en outre propre à déterminer l'état de fonctionnement des capteurs du système de capteurs 26, et à éliminer les données provenant des capteurs du système de capteurs 26 qui sont non fonctionnels. Il est propre à déterminer un état pilote de bas niveau uniquement sur la base des capteurs du système de capteurs de surveillance 26 qui sont opérationnels.

Le deuxième module d'évaluation de plus bas niveau 72 comporte ici en correspondance avec chaque première machine à états 80, une deuxième machine à états 82 déterministe.

La deuxième machine à états 82 est propre à mettre en oeuvre un algorithme déterministe sur la base des deuxièmes données de surveillance reçues de la deuxième interface 52, et éventuellement de premières données de surveillance reçues de la première interface 50.

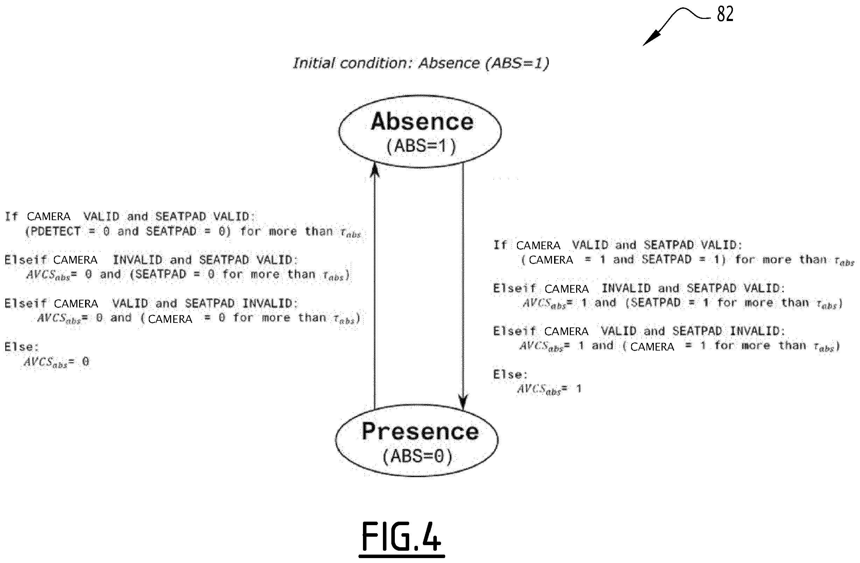

Dans l'exemple représenté sur la figure 4, la deuxième machine à états 82 est propre pour le ou chaque état pilote déterminé par l'unité de détermination 54, à déterminer un état pilote de bas niveau qui correspond à l'état pilote de haut niveau déterminé par la première machine à états 80 de la figure 3.

Elle est propre par exemple à déterminer un état d'absence ou un état de présence du pilote dans l'interface de commande sous forme d'un indicateur booléen ici désigné par ABS. Par défaut, l'état détecté est un état initial d'absence (« initial condition : Absence (ABS = 1) » ; « la condition initiale : Absence (ABS = 1) »).

Dans cet exemple, la deuxième machine 82 utilise des deuxièmes données de surveillance de plus bas niveau pour obtenir un indicateur booléen SEATPAD provenant de la jauge de pression 34 et un indicateur booléen CAMERA provenant du dispositif d'analyse 38 des images produites par la caméra 36. Elle utilise également l'indicateur booléen de détection du pilote AVCS_{abs} obtenu à partir des premières données de surveillance de haut niveau.

Partant d'un état d'absence ABS = 1, si les deuxièmes données de surveillance provenant de chaque capteur sont déclarées valides et si l'indicateur booléen SEATPAD et si l'indicateur booléen CAMERA indiquent simultanément une présence du pilote sur le siège (valeur égale à 1 sur la figure 4) pendant plus d'une durée déterminée τ_{abs} donnée (par exemple 5 secondes), alors l'état normal de présence est obtenu (ABS = 0).

Ceci est le cas également si l'indicateur booléen SEATPAD indique seul une présence du pilote sur le siège (valeur égale à 1 sur la figure 4) pendant plus d'une durée τ_{abs} donnée, la donnée de caméra étant invalide, et si l'indicateur booléen de détection du pilote AVCS_{abs} indique une présence du pilote. Ceci est le cas aussi si l'indicateur booléen CAMERA relative à la caméra indique seul une présence du pilote sur le siège (valeur égale à 1 sur la figure 4) pendant plus d'une durée τ_{abs} donnée, la donnée de siège étant invalide, et si l'indicateur booléen de détection du pilote AVCS_{abs} indique une présence du pilote.

Ceci est également le cas si l'indicateur booléen de détection du pilote AVCS_{abs} indique seul une présence du pilote lorsque les deux capteurs siège et caméra sont déclarés invalides.

Des conditions analogues permettent de détecter un état dégradé d'absence à partir de l'état normal de présence.

Le résultat donné par la deuxième machine à états 82 est donc propre à basculer entre un état pilote normal de bas niveau et au moins un état pilote dégradé de bas niveau, et à fournir ces données au module de consolidation 74.

Le module de consolidation 74 est propre à consolider les états pilotes de haut niveau et de bas niveau produits respectivement par le premier module d'évaluation de haut niveau 70 et par le deuxième module d'évaluation de plus bas niveau 72 pour produire l'état pilote consolidé.

Le module de consolidation 74 est propre à appliquer une logique de consolidation entre l'état pilote de haut niveau reçu du premier module d'évaluation 70 et l'état pilote de bas niveau reçu du deuxième module d'évaluation 72, dans la configuration d'activation du deuxième module d'évaluation de plus bas niveau 72.

La logique est par exemple une logique de type « OU ». Ainsi, si l'un au moins des états pilotes de haut niveau ou de bas niveau est respectivement un état dégradé de haut niveau ou un état dégradé de bas niveau, l'état pilote consolidé est alors basculé dans l'état dégradé.

Au contraire, lorsque le premier module d'évaluation 70 et le deuxième module d'évaluation 72 déterminent chacun un état normal, alors l'état pilote consolidé produit par le système de surveillance de l'état opérationnel d'un équipage 10 est un état pilote normal.

Ainsi, avec une architecture du type précité, qui prévoit une ségrégation des données de surveillance en fonction de leur niveau d'assurance de conception, entre un haut niveau et un plus bas niveau, chaque module d'évaluation 70, 72 prend en entrée les interfaces 50, 52 de niveau adapté à l'état pilote qu'il doit engendrer, et possède ses propres machines à états 80, 82 pour déterminer chacun un état pilote propre.

Le premier module d'évaluation de haut niveau 70 garantit un haut niveau d'assurance de conception de l'état pilote obtenu, puisqu'il utilise exclusivement en entrée des données de haut niveau d'assurance de conception, avec un algorithme déterministe. Ceci assure, lors d'une certification éventuelle du module de surveillance 10, la détection systématique des états dégradés principaux, en particulier de l'état d'absence et de l'état d'incapacité ou/et de sommeil.

Les performances de la première machine à états 80 sont cependant plus limitées, notamment en termes de délai de détection ou/et de type d'états détectés.

Le deuxième module d'évaluation de plus bas niveau 72 est quant à lui propre à assurer une efficacité opérationnelle du système, en accélérant et en améliorant le périmètre de détection, et en y intégrant des données de plus bas niveau, ce qui enrichit les états possibles pouvant être détectés.

La combinaison des états produits par les deux modules 70, 72, au sein du module de consolidation 74, couvre les objectifs que le système de surveillance 10 doit remplir, à savoir alerter l'équipage en cas d'incapacité, de sommeil ou d'absence du pilote, mais également une efficacité opérationnelle.

De préférence, la première interface de réception 50 reçoit, conjointement avec chaque première donnée, un niveau de validité associé à la première donnée, qui correspond à une quantification (éventuellement booléenne) de validité de la première donnée, en fonction par exemple de défauts ou de pannes du ou des systèmes produisant et transmettant la première donnée.

De même, la deuxième interface de réception 52 reçoit, conjointement avec chaque deuxième donnée, un niveau de validité associé à la deuxième donnée, qui correspond à une quantification (éventuellement booléenne) de validité de la deuxième donnée, en fonction par exemple de défauts ou de pannes du ou des systèmes produisant et transmettant la deuxième donnée.

Dans ce cas, le module d'évaluation de haut niveau 70 est propre à calculer un niveau de validité de haut niveau associé au ou à chaque état pilote de haut niveau déterminé, en fonction du niveau de validité des premières données utilisées.

Le module d'évaluation de bas niveau 72 est propre à calculer un niveau de validité de bas niveau associé à l'état pilote de bas niveau déterminé, en fonction du niveau de validité des deuxièmes données et éventuellement des premières données utilisées.

Le module de consolidation 74 est propre à calculer un niveau de validité consolidé de l'état pilote consolidé, sur la base des niveaux de validité respectivement de haut niveau et de bas niveau, en prenant par exemple le maximum, la moyenne, ou une pondération des niveaux de validité respectivement de haut niveau et de bas niveau.

Avantageusement, les algorithmes mis en oeuvre par les modules d'évaluation de haut niveau et de bas niveau 70, 72 sont aptes à prendre en compte les niveaux de validité des premières données et des deuxièmes données, pour adapter les logiques de détermination des états pilotes de haut et bas niveau. Ceci les rend robustes à la perte de capteurs ou de données d'entrées.

Avantageusement, le module d'évaluation de haut niveau 70 est propre à calculer un niveau de confiance de haut niveau associé au ou à chaque état pilote de haut niveau déterminé. Le module d'évaluation de bas niveau 72 est propre à calculer un niveau de confiance de bas niveau associé à l'état pilote de bas niveau déterminé.

Chaque niveau de confiance est par exemple obtenu à partir d'une table, et/ou d'un algorithme de calcul.

Le module de consolidation 74 est propre à calculer un niveau de confiance consolidé sur la base des niveaux de confiance respectivement de haut niveau et de bas niveau, en prenant par exemple le maximum, la moyenne, ou une pondération des niveaux de confiance respectivement de haut niveau et de bas niveau.

Le commutateur 76 est propre à être piloté depuis l'interface homme machine 62 pour basculer le module de consolidation 74 entre la configuration d'activation du deuxième module d'évaluation de plus bas niveau 72 et la configuration d'inactivation du deuxième module d'évaluation de plus bas niveau 72.

Dans la configuration d'activation, l'état pilote est déterminé à partir des états pilotes de haut niveau et de bas niveau par le module de consolidation 74, alors que dans la configuration inactive, seuls les états pilotes de haut niveau sont pris en compte par le module de consolidation 74 pour déterminer le ou chaque état pilote.

La présence du commutateur 76 fiabilise le système 10. Lorsque tous les capteurs fonctionnent, le système 10 utilise les données de tous les capteurs (dont les données de bas niveau d'assurance de conception), qui agrandissent le périmètre fonctionnel du système 10 et sont plus réactifs que les systèmes produisant les données de haut niveau d'assurance de conception, qui sont plus sûrs. Si jamais les capteurs produisant les données de bas niveau d'assurance de conception commencent à perturber le système 10 du fait de leur plus bas niveau d'assurance de conception, l'équipage peut les couper manuellement, pour recentrer le système 10 seulement sur les données de haut niveau d'assurance de conception. Même si le système 10 est alors plus limité fonctionnellement et en réactivité, il reste sûr et fiable, cohérent des exigences de certification. Comme on le décrira plus bas, le besoin de passer d'une configuration à l'autre est détectable par le pilote, en regardant par exemple l'afficheur 56.

Le gestionnaire d'affichage 58 comporte par exemple un processeur, et au moins une mémoire destinée à recevoir des modules logiciels propres à être exécutés par le processeur pour effectuer des fonctions.

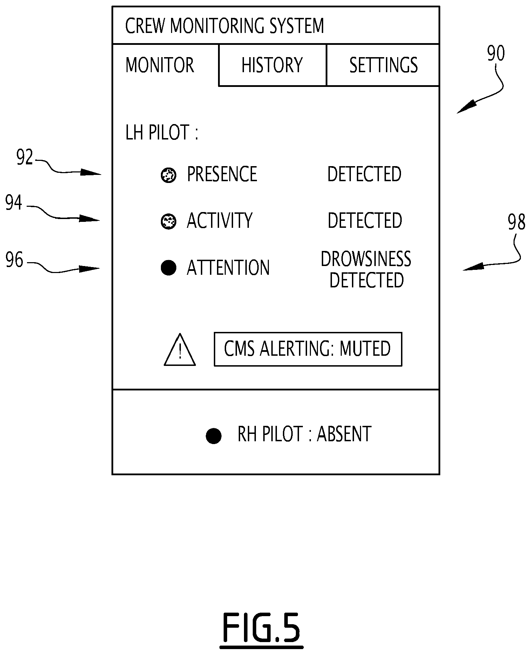

Il est propre à récupérer l'état pilote déterminé par le module de consolidation de l'unité de détermination 54, en particulier un état normal et/ou un état dégradé d'absence, d'incapacité ou/et de sommeil, de somnolence ou d'inattention. Il est propre à engendrer et à afficher sur l'afficheur 56 au moins une première fenêtre 90 de surveillance des états pilotes, dont un exemple est illustré sur la figure 5.

La fenêtre de surveillance 90 comporte par exemple un premier indicateur 92 indiquant la présence du pilote (« DETECTED » signifiant que le pilote est détecté), un deuxième indicateur 94 précisant si une activité du pilote est observée, résultant de l'absence d'état d'incapacité ou/et de sommeil, et un troisième indicateur 96 indiquant le niveau de vigilance du pilote, en particulier à l'aide d'un message 98 qui permet de déterminer si une somnolence (« DROWSINESS ») ou une inattention est détectée.

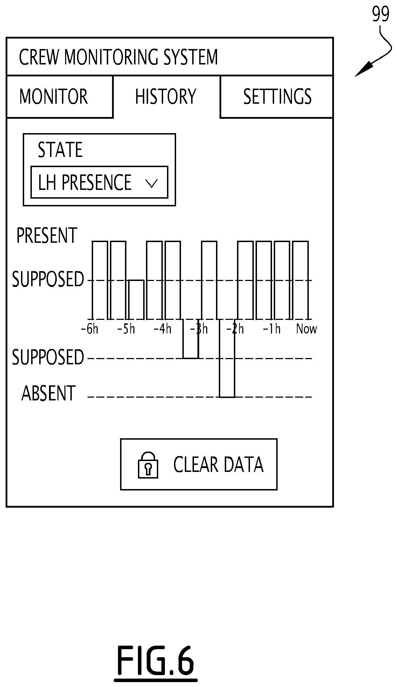

Le gestionnaire d'affichage 58 est propre en outre à engendrer avantageusement une deuxième fenêtre 99 pour récupérer l'historique de chacun des états pilotes au cours du temps. Par exemple sur la figure 6, l'historique de présence du pilote à son siège au cours du temps (« HISTORY ») est affiché sous forme d'un histogramme indiquant la présence (« PRESENT »), la présence supposée (« SUPPOSED »), l'absence supposée et l'absence (« ABSENT ») du pilote, le niveau de certitude provenant du niveau de confiance associée à l'état consolidé issu du module de consolidation 74.

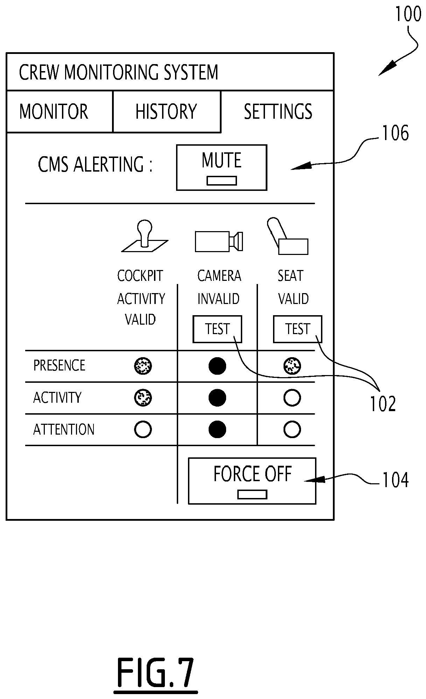

Le gestionnaire d'affichage 58 est en outre propre à afficher une fenêtre 100 de contrôle du système de surveillance de l'état opérationnel d'un équipage 10, visible sur la figure 7.

La fenêtre 100 comporte des boutons 102 de test des différents capteurs 28, 30 du système de capteurs 26, un système de pilotage 104 de la commande 76 pour désactiver le deuxième module d'évaluation de plus bas niveau 72, en cas de dysfonctionnement observé des capteurs 28, 30, et éventuellement un bouton 106 de mise en silence des générateurs d'informations et/ou d'alarme 60.

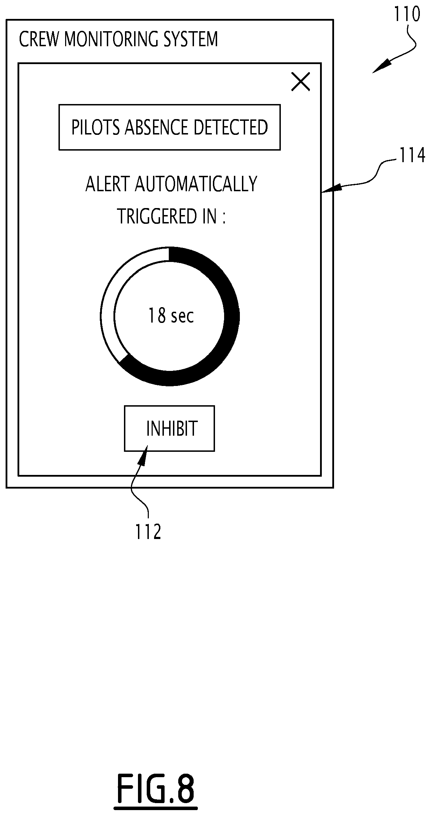

En référence à la figure 8, le gestionnaire d'affichage 58 est propre en outre à afficher, en cas de détection d'un état pilote dégradé, une fenêtre 110 de sollicitation du pilote à laquelle le pilote doit répondre pour confirmer ou infirmer l'état dégradé supposé détecté par le système. Cette fenêtre comporte un bouton 112 d'acquittement, et un compteur 114 qui précise le temps restant avant que le générateur d'informations et/ou d'alarme 60 engendre une alarme.

Dans le cas où l'état pilote est un état d'absence, d'incapacité et/ou de sommeil, le générateur d'informations et/ou d'alarme 60 est propre à émettre une information, par exemple une notification ou un message, voire une alarme dans l'interface de commande à l'attention du second pilote ou d'un autre membre d'équipage. Cette alarme est par exemple une alarme visuelle ou/et auditive ou/et vibratoire. Ces alarmes sont toujours engendrées dès lors qu'un état d'absence, d'incapacité et/ou de sommeil est constatée.

Par ailleurs, dans le cas d'un état de somnolence ou d'inattention, une alarme visuelle ou auditive, ou encore une alarme vibratoire est engendrée, à l'attention du pilote concerné.

Le fonctionnement du système de surveillance de l'état opérationnel d'un équipage 10 selon l'invention va maintenant être décrit.

Le système 10 est destiné à fonctionner de préférence durant toute la durée du vol.

Une fois le système de surveillance de l'état opérationnel d'un équipage 10 activé, la première interface 50 de réception de premières données de haut niveau d'assurance de conception reçoit en continu des premières données de surveillance provenant par exemple de l'unité centrale d'avionique 16 sur le bus de données 25. Ces premières données de surveillance traduisent notamment l'activité du pilote sur les commandes d'avionique et présentent un haut niveau d'assurance de conception.

De même, les capteurs 28, 30 du système de capteurs de surveillance 26 sont activés pour détecter la présence éventuelle d'un pilote sur son siège à l'aide de la jauge de pression 34, et/ou pour filmer le pilote, à l'aide du système de caméra 30, les données de la caméra 36 étant analysées en continu par le dispositif d'analyse d'images 38.

Ces deuxièmes données de surveillance, de plus bas niveau d'assurance de conception, sont transmises à la deuxième interface de réception 52.

À tout instant, le premier module d'évaluation 70 reçoit exclusivement les premières données provenant de la première interface 50, et met en oeuvre, pour chaque état pilote surveillé, l'algorithme déterministe présent dans la première machine à états 80 correspondant à l'état pilote surveillé.

Pour chaque état pilote surveillé, la mise en oeuvre de l'algorithme, sur la base des premières données, conduit à déterminer un état pilote de haut niveau, qui peut être un état normal de haut niveau ou un état dégradé de haut niveau.

Simultanément, dans une configuration d'activation, le deuxième module d'évaluation de plus bas niveau 72 reçoit les deuxièmes données provenant de la deuxième interface 52, éventuellement accompagnées de premières données provenant de la première interface 50.

Pour chaque état pilote surveillé, il met alors en oeuvre l'algorithme déterministe de la deuxième machine à états 82.

La deuxième machine à états 82 est ainsi apte à produire, pour chaque état pilote surveillé, un état pilote de bas niveau, qui peut être un état normal de bas niveau, ou un état dégradé de bas niveau.

Le module de consolidation 74 reçoit alors, pour chaque état pilote surveillé, l'état pilote de haut niveau et l'état pilote de bas niveau et détermine un état pilote consolidé.

Si chacun des états pilotes de haut niveau et de bas niveau est un état normal, le module de consolidation 74 détermine que l'état pilote consolidé est un état normal.

Si au contraire, l'un ou moins des états pilote de bas niveau ou de haut niveau est un état dégradé, le module de consolidation 74 détermine que l'état pilote consolidé est un état dégradé.

Chaque état pilote attribué par le module de consolidation 74 est ensuite transmis au gestionnaire d'affichage 58, pour engendrer et afficher la fenêtre de surveillance 90 avec le ou chaque état pilote déterminé à l'instant donné.

Par ailleurs, chaque état pilote est également transmis au générateur d'informations et/ou d'alarme 60. En cas d'état pilote dégradé, le générateur d'informations et/ou d'alarme 60 active tout d'abord le gestionnaire d'affichage 58 pour engendrer et afficher la fenêtre 100 de sollicitation au pilote, puis en l'absence de réponse du pilote à cette fenêtre 100, active les alarmes définies ci-dessus.

Si le pilote estime que l'alarme engendrée est injustifiée ou est une gêne, il active la fenêtre de réglage de paramètres 99. Il peut alors tester le bon fonctionnement des capteurs de détection 28 ou du système de caméra 30 à l'aide des boutons 102. Il peut également actionner la commande 76 pour désactiver le deuxième module d'évaluation de plus bas niveau 72 et empêcher un état pilote de bas niveau d'être transmis au module de consolidation 74. Le module de consolidation 74 détermine alors l'état pilote uniquement sur la base de l'état pilote de haut niveau.

Le système de surveillance 10 selon l'invention détecte donc de manière très fiable via le premier module d'évaluation de haut niveau 70, dans un délai déterminé (par exemple une durée typique d'environ 5 minutes), qu'un pilote en fonction est absent de son poste ou présente une incapacité ou un sommeil profond. Ce délai maximal est réduit grâce à la présence du module d'évaluation de plus bas niveau 72, qui détecte de manière beaucoup plus sensible des états dégradés du pilote, même si les capteurs qui sont utilisés pour cette détection sont de niveau d'assurance de conception moindre.

Ainsi, en ségrégant les données reçues par l'unité de détermination pilote 54 entre les premières données de haut niveau d'assurance de conception, et les deuxièmes données de plus bas niveau d'assurance de conception, il est possible de répondre au double objectif de sécurité et de réactivité du système de surveillance 10.

Le système de surveillance 10 selon l'invention présente un niveau de sécurité très élevé grâce à la ségrégation en deux niveaux de détection, un fiable mais de performance réduite, et un plus réactif, mais potentiellement générateur de nuisance ou de perturbation, et la capacité à isoler ce second niveau.

Le système de surveillance 10 selon l'invention est par ailleurs réactif, puisque le deuxième module d'évaluation de plus bas niveau 72 reçoit des données plus riches que celles qui sont utilisées par le premier module d'évaluation 70, permettant une détection plus fine des états dégradés et souvent plus rapide.

En tout état de cause, même si les deuxièmes données de plus bas niveau sont de plus bas niveau d'assurance de conception que les premières données de haut niveau, elles peuvent être exclues automatiquement par le module d'évaluation de plus bas niveau 72 lorsqu'un capteur qui produit ces données est détecté comme non-opérationnel. Elles peuvent être également volontairement écartées par le pilote, qui peut utiliser la commande 76 pour désactiver la prise en compte de l'état pilote de bas niveau.

Ainsi, le système de surveillance 10 est suffisamment fiable pour être certifiable, tout en étant réactif pour être opérationnel auprès des pilotes lorsque tout fonctionne de manière nominale, situation usuelle.

Le système de surveillance 10 est capable de fonctionner de manière dégradée en sélectionnant les capteurs disponibles restants, tout en offrant à l'utilisateur la possibilité de supprimer la prise en compte des données produites par les capteurs de faible niveau d'assurance de conception, si ceux-ci donnent des résultats erronés.

En outre, le système de surveillance 10 autorise l'intégration parmi les données de surveillance de haut niveau de conception, via l'interface 50 de données provenant de capteurs de niveau d'assurance de conception améliorée, par exemple des capteurs de siège améliorés. Il autorise également la prise en compte de nouveaux capteurs en plus du capteur de détection 28 et du système de caméra 30, même si leur fiabilité est moindre.

De même, le système de surveillance 10 peut utiliser des données du bus avionique 19 de plus bas niveau de conception qui sont récupérées par la deuxième interface 52.

Dans une variante (non représentée), l'algorithme mis en oeuvre par le deuxième module d'évaluation de plus bas niveau 72 n'est pas un algorithme déterministe, mais est par exemple un algorithme fonctionnant par apprentissage. L'algorithme mis en oeuvre par le premier module d'évaluation de haut niveau 70 reste un algorithme déterministe.

Dans une autre variante, l'état pilote consolidé obtenu pour le ou chaque état pilote déterminé, est propre à passer d'un état normal à une pluralité d'états dégradés de niveaux différents.

## Revendications

1. Système (10) de surveillance de l'état opérationnel d'un équipage d'aéronef comprenant :
- une première interface (50) de réception de premières données de surveillance d'équipage, les premières données de surveillance présentant un haut niveau d'assurance de conception ;
- une deuxième interface (52) de réception de deuxièmes données de surveillance d'équipage, les deuxièmes données de surveillance présentant un niveau d'assurance de conception plus bas que les premières données de surveillance ;
- une unité (54) de détermination d'au moins un état pilote en fonction des premières données de surveillance de haut niveau d'assurance de conception reçues de la première interface de réception (50), et des deuxièmes données de surveillance de plus bas niveau d'assurance de conception reçues de la deuxième interface de réception (52), **caractérisée en ce que** l'unité de détermination (54) comporte, pour le ou chaque état pilote surveillé :
• un premier module (70) d'évaluation de haut niveau, propre à mettre en oeuvre un premier algorithme déterministe pour obtenir un état pilote de haut niveau à partir des premières données de surveillance de haut niveau d'assurance de conception, sans tenir compte des deuxièmes données de surveillance de plus bas niveau d'assurance de conception ;
• un deuxième module (72) d'évaluation de plus bas niveau, propre à déterminer un état pilote de bas niveau, à partir d'au moins les deuxièmes données de surveillance de plus bas niveau d'assurance de conception ;
• un module (74) de consolidation, propre à déterminer un état pilote consolidé, l'état pilote consolidé étant obtenu, dans une configuration active du deuxième module d'évaluation de plus bas niveau (72), en fonction de l'état pilote de haut niveau et de l'état pilote de bas niveau, et étant obtenu, dans une configuration inactive du deuxième module d'évaluation de plus bas niveau (72), en fonction de l'état pilote de haut niveau, sans tenir compte de l'état pilote de bas niveau.

2. Système (10) selon la revendication 1, dans lequel l'état pilote consolidé déterminé par le module de consolidation (74) est propre à passer d'un état normal à au moins un état dégradé, l'état pilote de haut niveau étant propre à passer d'un état normal de haut niveau à au moins un état dégradé de haut niveau, l'état pilote de bas niveau étant propre à passer d'un état normal de bas niveau à un état dégradé de bas niveau, le module de consolidation (74) étant propre à établir un état pilote dégradé lorsque le premier module d'évaluation de haut niveau (70) détermine un état pilote dégradé de haut niveau ou/et lorsque le deuxième module de plus bas niveau (72) détermine un état pilote dégradé de bas niveau.

3. Système (10) selon l'une quelconque des revendications précédentes, dans lequel le premier module d'évaluation de haut niveau (70) comporte une première machine à états finis (80) mettant en oeuvre le premier algorithme déterministe.

4. Système (10) selon l'une quelconque des revendications précédentes, dans lequel le deuxième module d'évaluation de plus bas niveau (72) est propre à mettre en oeuvre un deuxième algorithme, de préférence un deuxième algorithme déterministe, et éventuellement, dans lequel le deuxième module d'évaluation de plus bas niveau (72) comporte une deuxième machine à états finis (82) mettant en oeuvre le deuxième algorithme.

5. Système (10) selon l'une quelconque des revendications précédentes, dans lequel la première interface de réception (50) est propre à recevoir, conjointement avec chaque première donnée, un niveau de validité associé à la première donnée, la deuxième interface de réception (52) étant propre à recevoir, conjointement avec chaque deuxième donnée, un niveau de validité associé à la deuxième donnée, le module d'évaluation de haut niveau (70) et le module d'évaluation de bas niveau (72) étant aptes à prendre en compte les niveaux de validité des premières données et des deuxièmes données, pour déterminer les états pilotes de haut niveau et de bas niveau ou/et pour déterminer un niveau de validité associé à l'état pilote considéré.

6. Système (10) selon l'une quelconque des revendications précédentes, dans lequel le deuxième module d'évaluation de plus bas niveau (72) est propre à recevoir des premières données de surveillance de haut niveau d'assurance de conception de la première interface de réception (50), le deuxième module d'évaluation de plus bas niveau (72) étant propre à obtenir l'état pilote de bas niveau en fonction de deuxièmes données de surveillance de plus bas niveau d'assurance de conception obtenues à partir de la deuxième interface (52) et de premières données de surveillance de haut niveau d'assurance de conception obtenues à partir de la première interface (50).

7. Système (10) selon l'une quelconque des revendications précédentes, dans lequel le premier module d'évaluation de haut niveau (70) est propre à calculer un niveau de confiance de haut niveau associé au ou à chaque état pilote de haut niveau déterminé, le deuxième module d'évaluation de bas niveau (72) étant propre à calculer un niveau de confiance de bas niveau associé à l'état pilote de bas niveau déterminé, le module de consolidation (74) étant propre à calculer un niveau de confiance consolidé associé à l'état pilote consolidé, sur la base du niveau de confiance de haut niveau et du niveau de confiance de bas niveau.

8. Système (10) selon l'une quelconque des revendications précédentes, dans lequel l'état pilote consolidé est choisi parmi un état de présence à son poste de travail, un état de capacité d'opération de l'aéronef, en particulier d'incapacité ou/et de sommeil, un état de vigilance, notamment de somnolence, de distraction et/ou d'inattention, un état de surcharge de travail, un état de surcharge mentale, un état de stress, un état de niveau de conscience de la situation présente, un état d'engagement dans une tâche, un état de niveau d'activité physique, et/ou un état de niveau de cohérence et de pertinence de l'activité pilote avec la mission.

9. Système (10) selon l'une quelconque des revendications précédentes, dans lequel l'unité de détermination (54) comporte une commande (76) d'inactivation du module d'évaluation de plus bas niveau (72), accessible à un membre d'équipage, propre à passer le module d'évaluation de plus bas niveau (72) de la configuration active à la configuration inactive.

10. Système (10) selon l'une quelconque des revendications précédentes, dans lequel la première interface de réception (50) et/ou la deuxième interface de réception (52) est propre à recevoir des données d'un système d'avionique (14), ou/et des données d'interaction du membre d'équipage avec une interface homme/machine (19) du système d'avionique (14).

11. Système (10) selon l'une quelconque des revendications précédentes, dans lequel la première interface de réception (50) et/ou la deuxième interface de réception (52) est propre à obtenir des données de capteurs de surveillance d'états du membre d'équipage, les capteurs de surveillance d'états du membre d'équipage comprenant éventuellement un capteur (28) de détection de présence d'un membre d'équipage sur le siège, un capteur (30) optique de détection présentant un champ de vision en regard d'un siège du membre d'équipage, notamment une caméra fonctionnant dans le domaine visible, proche infrarouge, thermique, et/ou une caméra temps de vol, en 2D et/ou en 3D, ou/et au moins un capteur (40, 42) de détection de paramètres physiologiques, notamment un capteur ECG, un capteur de détection de respiration, un capteur EEG, un capteur FNIR, un capteur de mesure de données physiologiques logé dans une montre connectée, un bracelet connecté ou dans un vêtement connecté.

12. Système (10) selon l'une quelconque des revendications précédentes, comportant un afficheur (56), et un gestionnaire d'affichage (58) sur l'afficheur (56), propre à générer et à afficher sur l'afficheur (56) une fenêtre (90) de surveillance d'états, illustrative du ou de chaque état pilote consolidé déterminé par l'unité de détermination (54).

13. Système (10) selon l'une quelconque des revendications précédentes, comportant un générateur d'informations, notamment de notification ou de message, et/ou d'alarme (60), propre à générer au moins une information, notamment une notification ou un message, et/ou au moins une alarme lorsqu'un état pilote consolidé déterminé par l'unité de détermination (54) est dans un état dégradé.

14. Procédé de surveillance de l'état opérationnel d'un équipage d'aéronef, mis en oeuvre dans un système de surveillance (10), comprenant les étapes suivantes :
- réception, par une première interface (50) de réception du système de surveillance (10), de premières données de surveillance d'équipage, les premières données de surveillance présentant un haut niveau d'assurance de conception ;
- réception, par une deuxième interface (52) de réception du système de surveillance (10), de deuxièmes données de surveillance présentant un niveau d'assurance de conception plus bas que les premières données de surveillance ;
- détermination, par une unité (54) de détermination du système de surveillance, d'au moins un état pilote en fonction des premières données de surveillance de haut niveau d'assurance de conception reçues de la première interface de réception (50), et des deuxièmes données de surveillance de plus bas niveau d'assurance de conception reçues de la deuxième interface de réception (52), la détermination comportant, pour le ou chaque état pilote surveillé :
• mise en oeuvre d'un algorithme déterministe par un premier module (70) d'évaluation de haut niveau de l'unité de détermination (54) pour obtenir un état pilote de haut niveau à partir des premières données de surveillance de haut niveau d'assurance de conception, sans tenir compte des deuxièmes données de surveillance de plus bas niveau d'assurance de conception ;
• détermination par un deuxième module (72) d'évaluation de plus bas niveau de l'unité de détermination (54), d'un état pilote de bas niveau, à partir d'au moins les deuxièmes données de surveillance de plus bas niveau d'assurance de conception ;
• détermination d'un état pilote consolidé par un module (74) de consolidation de l'unité de détermination (54), l'état pilote consolidé étant obtenu, dans une configuration active du deuxième module d'évaluation de plus bas niveau (72), en fonction de l'état pilote de haut niveau et de l'état pilote de bas niveau, et étant obtenu, dans une configuration inactive du deuxième module d'évaluation de plus bas niveau (72), en fonction de l'état pilote de haut niveau, sans tenir compte d'un état pilote de bas niveau.

15. Procédé selon la revendication 14, dans lequel lorsque l'état pilote de haut niveau passe d'un état normal de haut niveau à un état dégradé de haut niveau, et/ou lorsque l'état pilote de bas niveau correspondant à l'état pilote de haut niveau passe d'un état normal de bas niveau à un état dégradé de bas niveau, le module de consolidation (74) établit que l'état pilote consolidé passe d'un état normal à un état dégradé.

## Patentansprüche

1. System (10) zur Überwachung des Einsatzzustands einer Flugzeugbesatzung, umfassend:
- eine erste Empfangsschnittstelle (50) von ersten Überwachungsdaten der Besatzung, wobei die ersten Überwachungsdaten ein hohes Design-Assurance-Level aufweisen;
- eine zweite Empfangsschnittstelle (52) von zweiten Überwachungsdaten der Besatzung, wobei die zweiten Überwachungsdaten ein niedrigeres Design-Assurance-Level als die ersten Überwachungsdaten aufweisen;
- eine Einheit (54) zum Bestimmen mindestens eines Pilotenzustands in Abhängigkeit von den von der ersten Empfangsschnittstelle (50) empfangenen ersten Überwachungsdaten mit einem hohen Design-Assurance-Level und von den zweiten von der zweiten Empfangsschnittstelle (52) empfangenen Überwachungsdaten mit einem niedrigeren Design-Assurance-Level, **dadurch gekennzeichnet, dass** die Bestimmungseinheit (54) für den oder jeden überwachten Pilotenzustand umfasst:
• ein erstes High-Level-Evaluierungsmodul (70), das imstande ist, einen ersten deterministischen Algorithmus auszuführen, um einen High-Level-Pilotenzustand aus den ersten Überwachungsdaten mit einem hohen Design-Assurance-Level zu erhalten, ohne die zweiten Überwachungsdaten mit einem niedrigeren Design-Assurance-Level zu berücksichtigen;
• ein zweites Lower-Level-Evaluierungsmodul (72), das imstande ist, einen Low-Level-Pilotenzustand aus mindestens den zweiten Überwachungsdaten mit einem niedrigeren Design-Assurance-Level zu bestimmen;
• ein Konsolidierungsmodul (74), das imstande ist, einen konsolidierten Pilotenzustand zu bestimmen, wobei der konsolidierte Pilotenzustand in einer aktiven Konfiguration des zweiten Lower-Level-Evaluierungsmoduls (72) in Abhängigkeit von dem High-Level-Pilotenzustand und dem Low-Level-Pilotenzustand erhalten wird, und in einer inaktiven Konfiguration des zweiten Lower-Level-Evaluierungsmoduls (72) in Abhängigkeit von dem High-Level-Pilotenzustand ohne Berücksichtigung des Low-Level-Pilotenzustands erhalten wird.

2. System (10) nach Anspruch 1, wobei der durch das Konsolidierungsmodul (74) bestimmte konsolidierte Pilotenzustand imstande ist, aus einem normalen Zustand in mindestens einen verschlechterten Zustand zu wechseln, wobei der High-Level-Pilotenzustand imstande ist, aus einem normalen High-Level-Zustand in mindestens einen verschlechterten High-Level-Zustand zu wechseln, wobei der Low-Level-Pilotenzustand imstande ist, aus einem normalen Low-Level-Zustand in einen verschlechterten Low-Level-Zustand zu wechseln, wobei das Konsolidierungsmodul (74) imstande ist, einen verschlechterten Pilotenzustand festzulegen, wenn das erste High-Level-Evaluierungsmodul (70) einen verschlechterten High-Level-Pilotenzustand bestimmt, oder/und wenn das zweite Lower-Level-Modul (72) einen verschlechterten Low-Level-Pilotenzustand bestimmt.

3. System (10) nach einem der vorhergehenden Ansprüche, wobei das erste High-Level-Evaluierungsmodul (70) eine erste Finite-State-Maschine (80) aufweist, die den ersten deterministischen Algorithmus ausführt.

4. System (10) nach einem der vorhergehenden Ansprüche, wobei das zweite Lower-Level-Evaluierungsmodul (72) imstande ist, einen zweiten Algorithmus, vorzugsweise einen zweiten deterministischen Algorithmus, auszuführen, und gegebenenfalls wobei das zweite Lower-Level-Evaluierungsmodul (72) eine zweite Finite-State-Maschine (82) aufweist, die den zweiten Algorithmus ausführt.

5. System (10) nach einem der vorhergehenden Ansprüche, wobei die erste Empfangsschnittstelle (50) imstande ist, gemeinsam mit jedem ersten Datenwert ein Validitätslevel zu empfangen, das dem ersten Datenwert zugeordnet ist, wobei die zweite Empfangsschnittstelle (52) imstande ist, gemeinsam mit jedem zweiten Datenwert ein Validitätslevel zu empfangen, das dem zweiten Datenwert zugeordnet ist, wobei das High-Level-Evaluierungsmodul (70) und das Lower-Level-Evaluierungsmodul (72) imstande sind, die Validierungslevel der ersten Daten und der zweiten Daten zu berücksichtigen, um die High-Level- und Low-Level-Pilotenzustände zu bestimmen und/oder um ein Validierungslevel zu bestimmen, das dem entsprechenden Pilotenzustand zugeordnet ist.

6. System (10) nach einem der vorhergehenden Ansprüche, wobei das zweite Lower-Level-Evaluierungsmodul (72) imstande ist, erste High-Level-Design-Assurance-Level-Überwachungsdaten von der ersten Empfangsschnittstelle (50) zu empfangen, wobei das zweite Lower-Level-Evaluierungsmodul (72) imstande ist, den Low-Level-Pilotenzustand in Abhängigkeit von zweiten Lower-Level-Design-Assurance-Level-Überwachungsdaten, die von der zweiten Schnittstelle (52) erhalten werden, und erste High-Level-Design-Assurance-Level-Überwachungsdaten, die von der ersten Schnittstelle (50) erhalten werden, zu erhalten.

7. System (10) nach einem der vorhergehenden Ansprüche, wobei das erste High-Level-Evaluierungsmodul (70) imstande ist, ein High-Level-Konfidenzlevel zu berechnen, das dem oder jeden bestimmten High-Level-Pilotenzustand zugeordnet ist, wobei das zweite Low-Level-Evaluierungsmodul (72) imstande ist, ein Low-Level-Konfidenzlevel zu berechnen, das dem bestimmten Low-Level-Pilotenzustand zugeordnet ist, wobei das Konsolidierungsmodul (74) imstande ist, ein konsolidiertes Konfidenzlevel auf der Basis des High-Level-Konfidenzlevels und des Low-Level-Konfidenzlevels zu berechnen, das dem konsolidierten Pilotenzustand zugeordnet ist.

8. System (10) nach einem der vorhergehenden Ansprüche, wobei der konsolidierte Pilotenzustand aus einem Zustand der Anwesenheit an seinem Arbeitsplatz, einem Zustand der Fähigkeit zur Bedienung des Luftfahrzeugs, insbesondere der Unfähigkeit oder/und des Müdigkeits, einem Zustand der Aufmerksamkeit, insbesondere der Schläfrigkeit, der Ablenkung und/oder der Unaufmerksamkeit, einem Zustand der Arbeitsüberlastung, einem Zustand der mentalen Überlastung, einem Stresszustand, einem Zustand, sich der gegenwärtigen Situation bewusst zu sein, einem Zustand des Engagements für einer Aufgabe, einem Niveauzustand der körperlichen Aktivität und/oder einem Niveauzustand der Kohärenz und Relevanz der Pilotenaktivität mit der Mission ausgewählt ist.

9. System (10) nach einem der vorhergehenden Ansprüche, wobei die Bestimmungseinheit (54) einen Inaktivierungsbefehl (76) des Lower-Level-Evaluierungsmoduls (72) aufweist, der für ein Besatzungsmitglied erreichbar ist, der imstande ist, das Lower-Level-Evaluierungsmodul (72) aus der aktiven Konfiguration in die inaktive Konfiguration umzuschalten.

10. System (10) nach einem der vorhergehenden Ansprüche, wobei die erste Empfangsschnittstelle (50) und/oder die zweite Empfangsschnittstelle (52) imstande ist, Daten von einem Avioniksystem (14) oder/und Interaktionsdaten des Besatzungsmitglieds mit einer Mensch-Maschine-Schnittstelle (19) des Avioniksystems (14) zu erhalten.

11. System (10) nach einem der vorhergehenden Ansprüche, wobei die erste Empfangsschnittstelle (50) und/oder die zweite Empfangsschnittstelle (52) imstande ist, Daten von Sensoren zur Überwachung von Zuständen des Besatzungsmitglieds zu erhalten, wobei die Sensoren zur Überwachung von Zuständen des Besatzungsmitglieds gegebenenfalls einen Sensor (28) zur Detektion der Anwesenheit eines Besatzungsmitglieds auf dem Sitz, einen optischen Detektionssensor (30) mit einem Sichtfeld gegenüber einem Sitz des Besatzungsmitglieds aufweisen, insbesondere eine Kamera, die im sichtbaren Bereich, im nahen Infrarotbereich, im thermischen Bereich und/oder eine Flugzeitkamera in 2D und/oder 3D oder/und mindestens einen Sensor (40, 42) zur Detektion von physiologischen Parametern, insbesondere einen EKG-Sensor, einen Sensor zur Detektion der Atmung, einen EEG-Sensor, einen RFID-Sensor, einen Sensor zur Messung physiologischer Daten, der in einer verbundenen Uhr, einem verbundenen Armband oder in einem verbundenen Kleidungsstück untergebracht ist, umfassen.

12. System (10) nach einem der vorhergehenden Ansprüche, das ein Display (56) und einen Displaymanager (58) auf dem Display (56) aufweist, der imstande ist, auf dem Display (56) ein Zustandsüberwachungsfenster (90) zu erzeugen und anzuzeigen, das den oder jeden konsolidierten Pilotenzustand illustriert, der von der Bestimmungseinheit (54) bestimmt wurde.

13. System (10) nach einem der vorhergehenden Ansprüche, das einen Informations-, insbesondere einen Benachrichtigungs- oder Nachrichten- und/oder Alarmerzeuger (60) aufweist, der imstande ist, mindestens eine Information, insbesondere eine Benachrichtigung oder eine Nachricht und/oder mindestens einen Alarm zu erzeugen, wenn ein von der Bestimmungseinheit (54) bestimmter konsolidierter Pilotenzustand in einem verschlechterten Zustand ist.

14. Verfahren zur Überwachung des Einsatzzustands einer Flugzeugbesatzung, das in einem Überwachungssystem (10) ausgeführt wird, das die folgenden Schritte umfasst:
- Empfangen, durch eine erste Empfangsschnittstelle (50) des Überwachungssystems (10), von ersten Überwachungsdaten der Besatzung, wobei die ersten Überwachungsdaten ein hohes Design-Assurance-Level aufweisen;
- Empfangen, von einer zweiten Empfangsschnittstelle (52) des Überwachungssystems (10), von zweiten Überwachungsdaten, die ein niedrigeres Design-Assurance-Level als die ersten Überwachungsdaten aufweisen;
- Bestimmen, durch eine Bestimmungseinheit (54) des Überwachungssystems, mindestens eines Pilotenzustands in Abhängigkeit von den von der ersten Empfangsschnittstelle (50) empfangenen ersten Überwachungsdaten mit einem hohen Design-Assurance-Level und von den zweiten von der zweiten Empfangsschnittstelle (52) empfangenen Überwachungsdaten mit einem niedrigeren Design-Assurance-Level, wobei die Bestimmung für den oder jeden überwachten Pilotenzustand aufweist:
• Ausführen eines deterministischen Algorithmus durch ein erstes High-Level-Evaluierungsmodul (70) der Bestimmungseinheit (54), um einen High-Level-Pilotenzustand aus den ersten Überwachungsdaten mit einem hohen Design-Assurance-Level zu erhalten, ohne die zweiten Überwachungsdaten mit einem niedrigeren Design-Assurance-Level zu berücksichtigen;
• Bestimmen, durch ein zweites Lower-Level-Evaluierungsmodul (72) der Bestimmungseinheit (54), eines Low-Level-Pilotenzustands aus mindestens den zweiten Überwachungsdaten mit einem niedrigeren Design-Assurance-Level;
• Bestimmen eines konsolidierten Pilotenzustands durch ein Konsolidierungsmodul (74) der Bestimmungseinheit (54), wobei der konsolidierte Pilotenzustand in einer aktiven Konfiguration des zweiten Lower-Level-Evaluierungsmoduls (72) in Abhängigkeit von dem High-Level-Pilotenzustand und dem Low-Level-Pilotenzustand erhalten wird, und in einer inaktiven Konfiguration des zweiten Lower-Level-Evaluierungsmoduls (72) in Abhängigkeit von dem High-Level-Pilotenzustand ohne Berücksichtigung des Low-Level-Pilotenzustands erhalten wird.

15. Verfahren nach Anspruch 14, wobei, wenn der High-Level-Pilotenzustand aus einem normalen High-Level-Zustand in einen verschlechterten High-Level-Zustand wechselt, und/oder wenn der Low-Level-Pilotenzustand, der dem High-Level-Pilotenzustand entspricht, aus einem normalen Low-Level-Zustand in einen verschlechterten Low-Level-Zustand wechselt, das Konsolidierungsmodul (74) festlegt, dass der konsolidierte Pilotenzustand aus einem normalen Zustand in einen verschlechterten Zustand wechselt.

## Claims

1. A system (10) for monitoring the operational state of an aircraft crew comprising:
- a first interface (50) for receiving first crew monitoring data, the first monitoring data having a high design assurance level;
- a second interface (52) for receiving second crew monitoring data, the second monitoring data having a lower design assurance level than the first monitoring data;
- a unit (54) for determining at least one pilot state based on the first high design assurance monitoring data received from the first receiving interface (50), and on the second lower design assurance monitoring data received from the second receiving interface (52), **characterised in that** the determination unit (54) comprises, for the or each monitored pilot state:
• a first high-level evaluation module (70), adapted to implement a first deterministic algorithm to obtain a high-level pilot state from the first high design assurance level monitoring data, regardless of the second lower design assurance level monitoring data;
• a second lower-level evaluation module (72), suitable for determining a low-level pilot state, from at least the second lower design assurance level monitoring data;
• a consolidation module (74) for determining a consolidated pilot state, wherein the consolidated pilot state is obtained in an active configuration of the second lower-level evaluation module (72) as a function of the high-level pilot state and the low-level pilot state, and is obtained in an inactive configuration of the second lower-level evaluation module (72) as a function of the high-level pilot state, without taking the low-level pilot state into account.

2. The system (10) according to claim 1, wherein the consolidated pilot state determined by the consolidation module (74) is suitable for changing from a normal state to at least one degraded state, the high-level pilot state being suitable for changing from a normal high-level state to at least one degraded high-level state, the low-level pilot state being suitable for changing from a normal low-level state to a degraded low-level state, the consolidation module (74) being adapted to establish a degraded pilot state when the first high-level evaluation module (70) determines a high-level degraded pilot state and/or when the second lower-level module (72) determines a low-level degraded pilot state.

3. The system (10) according to any of the preceding claims, wherein the first high-level evaluation module (70) comprises a first finite-state machine (80) implementing the first deterministic algorithm.

4. The system (10) according to any one of the preceding claims, wherein the second lower level evaluation module (72) is adapted to implement a second algorithm, preferably a second deterministic algorithm and optionally, wherein the second lower level evaluation module (72) comprises a second finite-state machine (80) implementing the second algorithm

5. The system (10) according to any one of the preceding claims, wherein the first receiving interface (50) is adapted to receive, together with each first data item, a validity level associated with the first data item, the second receiving interface (52) being suitable for receiving, together with each second data item, a validity level associated with the second data item, the high-level evaluation module (70) and the low-level evaluation module (72) being able to take into account the validity levels of the first data and of the second data in order to determine the high-level and low-level pilot states and/or to determine a validity level associated with the pilot state in question.

6. The system (10) according to any of the preceding claims, wherein the second lower-level evaluation module (72) is adapted to receive first high design assurance level monitoring data from the first receiving interface (50), the second lower-level evaluation module (72) being adapted to obtain the low-level pilot state based on second lower-level design assurance monitoring data obtained from the second interface (52) and on first high design assurance level monitoring data obtained from the first interface (50).

7. The system (10) according to any one of the preceding claims, wherein the first high level evaluation module (70) is adapted to calculate a high level confidence level associated with the or each determined high-level pilot state, the second low level evaluation module (72) being adapted to calculate a low level confidence level associated with the determined low-level pilot state, the consolidation module (74) being adapted to calculate a consolidated confidence level associated with the consolidated pilot state, based on the high-level confidence level and the low-level confidence level.

8. The system (10) according to any one of the preceding claims, wherein the consolidated pilot state is selected from a state of presence at his/her work station, a state of aircraft operation ability, in particular of incapacitation and/or of sleep, a state of alertness, in particular drowsiness, distraction and/or inattention, a state of work overload, a state of mental overload, a state of stress, a state of present situation awareness, a state of task engagement, a state of physical activity level, and/or a state of pilot activity consistency and relevance with the mission.

9. The system (10) according to any one of the preceding claims, wherein the determination unit (54) comprises a command (76) for deactivating the lower-level evaluation module (72), accessible to a crew member, suitable for switching the lower-level evaluation module (72) from the active configuration to the inactive configuration.

10. The system (10) according to any one of the preceding claims, wherein the first receiving interface (50) and/or the second receiving interface (52) is adapted to receive data from an avionics system (14), and/or data from the crew member's interaction with a human/machine interface (19) of the avionics system (14).

11. The system (10) according to any of the preceding claims, wherein the first receiving interface (50) and/or the second receiving interface (52) is adapted to obtain data of the sensors for monitoring crew member states, the sensors for monitoring crew member states optionally comprise a sensor (28) for detecting the presence of a crew member on the seat, a sensor (30) of optical detection with a field of view facing a seat of the crew member, in particular a camera operating in the visible, near-infrared, or thermal spectrum, and/or a 2D and/or 3D time-of-flight camera, and/or at least one sensor (40, 42) for detecting physiological parameters, in particular an ECG sensor, a breathing detection sensor, an EEG sensor, an FNIR sensor, a sensor for measuring physiological data housed in a connected watch, a connected bracelet or in a connected garment

12. The system (10) according to any one of the preceding claims, comprising a display (56), and a display manager (58) on the display (56), adapted to generate and display on the display (56) a status monitoring window (90) illustrative of the or each consolidated pilot state determined by the determination unit (54).

13. The system (10) according to any one of the preceding claims, comprising an information generator, in particular a notification or message generator, and/or an alarm generator (60), capable of generating at least one item of information, in particular a notification or message, and/or at least one alarm when a consolidated pilot state determined by the determination unit (54) is in a degraded state.

14. A method of monitoring the operational state of an aircrew, implemented in a monitoring system (10), comprising the following steps:
- receiving, via a first receiving interface (50) of the monitoring system (10), first crew monitoring data, the first monitoring data having a high design assurance level;
- receiving, via a second receiving interface (52) of the monitoring system (10), second monitoring data having a lower design assurance level than the first monitoring data;
- determining, via a determination unit (54) of the monitoring system, at least one pilot state based on the first high design assurance monitoring data received from the first receiving interface (50), and the second lower design assurance monitoring data received from the second receiving interface (52), the determination comprising, for the or each monitored pilot state:
• implementation of a deterministic algorithm by a first high-level evaluation module (70) of the determination unit (54) to obtain a high-level pilot state from the first high design assurance level monitoring data, regardless of the second lower-level design assurance monitoring data;
• determination of a low-level pilot state by a second lower-level evaluation module (72) of the determination unit (54) from at least the second lower-level design assurance monitoring data;
• determination of a consolidated pilot state by a consolidation module (74) of the determination unit (54), the consolidated pilot state being obtained in an active configuration of the second lower-level evaluation module (72) as a function of the high-level pilot state and the low-level pilot state, and is obtained in an inactive configuration of the second lower-level evaluation module (72) as a function of the high-level pilot state, without taking into account a low-level pilot state.

15. The method according to claim 14, wherein when the high-level pilot state changes from a normal high-level state to a degraded high-level state, and/or when the low-level pilot state corresponding to the high-level pilot state changes from a normal low-level state to a degraded low-level state, the consolidation module (74) determines that the consolidated pilot state has changed from a normal state to a degraded state.
